# EUROPEAN PATENT SPECIFICATION

(11) **EP 0 817 608 B1**
(45) Date of publication and mention of the grant of the patent: **20.12.2000**
(21) Application number: 96908066.2
(22) Date of filing: 15.03.1996
(51) Int. Cl.: A61K 7/50, A61K 7/48, A61K 7/06, C11D 3/37

(54) **LIQUID CLEANSER COMPOSITIONS**
FLÜSSIGREINIGERZUSAMMENSETZUNG
COMPOSITIONS NETTOYANTES LIQUIDES

(30) Priority: 29.03.1995 US 412803
(43) Date of publication of application: 14.01.1998
(73) Proprietor: UNILEVER PLC, London EC4P 4BQ (GB); UNILEVER N.V., 3013 AL Rotterdam (NL)
(72) Inventor: TSAUR, Liang, Shen, Norwood, NJ 07648 (US); SHEN, Shiji, Norwood, NJ 07648 (US); JOBLING, Margaret, Wirral, Merseyside L63 2HA (GB); ARONSON, Michael, Paul, County of Rockland, NY 10994 (US)
(74) Representative: Elliott, Peter William
(86) International application number: EP9601166
(87) International publication number: WO9629979

(56) References cited:
- EP-A- 0 093 601
- WO-A-94/03151
- WO-A-94/03152
- WO-A-95/34271
- WO-A-96/02224
- WO-A-96/02225
- US-A- 4 491 539
- US-A- 4 556 510
- US-A- 4 678 606
- US-A- 5 085 857

## Description

### Background of the Invention

The present invention relates to liquid cleanser or liquid personal wash compositions which are able to deliver cosmetic benefit agents to the skin. More particularly, the invention relates to such compositions comprising hydrogel compositions dispersed inside the liquid cleanser or liquid personal wash compositions (e.g., hydrogel "networks" present as macroscopic domains or discrete dispersed aqueous phases within said liquid compositions).

Liquid cleanser compositions comprising benefit agents are known in the art. The use of silicone oil droplets in such compositions to provide a moisturisation benefit, for example, is known.

A method for enhancing delivery of such benefit agents (e.g., silicone and vegetable oils) to the skin or hair involves the use of cationic hydrophilic polymers such as Polymer JR^{(R)} from Amerchol or Jaguar^{(R)} from Rhone Poulenc; as described in EP 93,602; WO 94/03152; and WO 94/03151. In these references, both the polymer and the, generally, small sized silicone particles are uniformly distributed throughout the entire liquid cleanser composition (i.e. there are no concentrated "pockets" of benefit agent).

In other references, hydrophilic polymers are themselves incorporated into liquid cleanser compositions to provide various benefits. For example, hydrophilic polymers such as guar gum, polyacrylamides, polyacrylic acid and polymer JR^{(R)} are disclosed in U.S. Patent Nos. 4,491,539; 4,556,510; 4,678,606; 5,002,680 and 4,917,823 as thickeners, lather modifying agents and skin feel agents. Again, in these references, the polymers are uniformly incorporated or distributed throughout the entire cleanser composition.

In no reference is there taught separate hydrogel particles or a dispersion acting as a type of structure to contain or entrap a benefit agent in a concentrated form in surfactant containing compositions.

Unexpectedly, applicants have found that, if the benefit agent is incorporated into a liquid cleanser composition in the form of an integral hydrogel carrier containing the benefit agent (i.e., a discrete aqueous phase trapping the benefit agent), then the level of deposition of the benefit agent on the skin or other substrate is significantly enhanced relative to a typical liquid or gel composition containing a cationic deposition agent such as a cationic guar which assists deposition of a benefit agent. The level of deposition is also higher than for compositions of the type having oil droplets dispersed throughout, particularly when small sized oil particles are used.

### Definition of the Invention

The present invention accordingly provides a liquid cleanser composition comprising:-
(1) 1 to 60% by wt. of an aqueous surfactant solution comprising at least one surfactant selected from anionic, nonionic, cationic, zwitterionic, and amphoteric surfactants and mixtures thereof; and
(2) 1 to 50% by wt. of a hydrogel composition comprising:
   (a) 0.5 to 50% by wt., based on the hydrogel composition, of at least one first water-soluble polymer, which is insolubilised in said aqueous surfactant solution; and
   (b) 0.2 to 25% by wt., based on the hydrogel composition, of at least a second water-soluble polymer which is soluble or dispersible in said aqueous surfactant solution; and
   (c) 5 to 65% by wt., based on the hydrogel composition, of a water-insoluble cosmetic benefit agent which is entrapped in a network formed by said first and second polymers, said benefit agent having a particle size in the range 0.2 to 200 µm; and
   said hydrogel composition comprises particles of a size with at least one dimension in the range of more than 25 and up to 10,000 µm.

The hydrogel dispersions/particles "trap" water-insoluble cosmetic benefit agents in a network formed by the first and second polymers forming the hydrogel. The polymer network which entraps the benefit agents disintegrates smoothly when rubbed on a substrate such as skin and thereby imparts desirable in-use characteristics (e.g., smooth, rich, creamier lather) to the composition.

The second water-soluble polymer or polymers is a property modifying polymer which is required (1) to help stabilise the benefit agent in the hydrogel composition and (2) to help provide the required gel strength of said hydrogel composition.

### BRIEF DESCRIPTION OF THE FIGURES

Figure 1 shows the effect of carrageenan concentration on the gel strength of the hydrogel composition. Carrageenan is an example of the first water-soluble polymer (i.e. component 2(a)).

Figure 2 shows the effect on gel strength when Acrysol, an acrylic polymer, is combined with carrageenan. Acrysol is an example of the second water-soluble polymer (i.e. 2(b) which is soluble in both water and the aqueous surfactant solution.

### Detailed Description of the Invention

The hydrogel compositions is uniformly dispersed and stably suspended in the liquid cleanser composition. It exists in the liquid cleanser composition as a separate polymer gel phase (i.e., it is formed when at least one polymer insolubilises when placed in the aqueous surfactant system of said liquid cleansers; a second property modifying polymer is also required) present as macroscopic domains (i.e., having a particle size more than 25 micrometers, preferably more than 100 micrometers, and up to 10,000 micrometers). Furthermore, these domains are capable of trapping cosmetic benefit agent which are water-insoluble within the gel structure, i.e., inside a web or network formed by the hydrogel noodles or particles. The hydrogel composition must have sufficient gel strength (generally provided by the first polymer) to retain the integrity of the discrete domains, and to trap and hold the insoluble benefit agent during storage and application of the liquid cleanser composition in use, however, the hydrogel must also be capable of disintegrating smoothly when the liquid cleanser composition is applied to and rubbed into the intended substrate, such as the skin, without causing any undesirable feeling of foreign matter or grittiness.

Hydrogel compositions according to the invention are generally prepared by first emulsifying or dispersing the water-insoluble benefit agent in an aqueous polymer solution to form an emulsion or dispersion. This aqueous polymer solution, which contains the dispersed benefit agent, will henceforth be designated as the "hydrogel precursor solution". The hydrogel precursor solution (containing both the first and second polymers defined as (2)(a) and (2)(b) above) is then added to and mixed with a surfactant system under such conditions that the hydrogel precursor solution becomes insoluble (due to component (2)(a)) and forms spherical, noodle shaped or, in some cases, irregular shaped hydrogel domains uniformly dispersed in said liquid cleanser composition. Hydrogel dispersion can be accomplished either in a batch-wise or a continuous process, depending on how the hydrogel precursor solution and the cleansing composition are mixed. A batch process such as an overhead mixer or a continuous process such as a two fluid coextrusion nozzle, an in-line injector or an in-line mixer can be used to make the hydrogel dispersion. The size of the hydrogel particles in the final liquid cleanser composition can be altered by changing the mixing speed, time and the mixing device. In general, by reducing the mixing speed and decreasing the mixing time, or using a mixing device that produces less shear force during mixing, hydrogels of larger particle size can be produced; the size of the hydrogel domains (e.g., particles), should have at least one dimension (e.g., the diameter) that is larger than 25 µm and preferably larger than 100 µm, more preferably larger than 200 µm, to allow optimal transfer of the hydrogel to the substrate during use. The diameter may be as high as 10,000 µm.

The compositions of the invention thus comprises three essential components: i) an aqueous based cleansing composition capable of suspending the hydrogel and maintaining its integrity; ii) a hydrogel forming polymer system (comprising a first polymer which insolubilizes upon contact with aqueous surfactant solution; and a second property modifying polymer which stabilizes the benefit agent and/or modifies gel strength); and iii) a water-insoluble cosmetic benefit agent. (Components (ii) and (iii) together form the "hydrogel" composition when formed in the base composition (i)). Each of these components is described in greater detail below.

As indicated above, the invention comprises a liquid cleanser composition comprising the hydrogel composition more fully defined below. The liquid composition itself is described in greater detail below.

### (i) Liquid Cleanser Composition

As noted, the invention relates to a liquid cleanser composition for personal cleansing in which a hydrogel incorporating a cosmetic benefit agent (i.e., containing the agent in a web produced by polymers (2)(a) and (2)(b)) is dispersed. The liquid cleanser composition comprises from about 1% to about 50%, preferably about 5% to about 25% by wt. of a hydrogel composition; and from about 1% to about 60% by wt. of surfactants selected from any known surfactants suitable for topical personal cleansing applications. The surfactants are selected from anionic, nonionic, cationic, amphoteric and zwitterionic surfactants and mixtures thereof.

It is critical that the liquid cleanser composition is capable of suspending the dispersed hydrogel phase to form a stable suspension. This requires that it has a sufficiently high low-shear viscosity to prevent settling (or creaming) of the hydrogel composition under the action of gravity during storage. This can be achieved by utilizing liquid compositions formulated to have a viscosity of preferably at least 1,500 cps, and most preferably at least 3,000 cps at a shear rate of 10 sec⁻¹ at 25°C. This viscosity is generally sufficient to stably suspend the hydrogel compositions without appreciable gravitational phase separation during storage. As the particle size of the hydrogel dispersion increases, liquids that have a higher viscosity are required to achieve adequate stability.

The viscosity of the liquid cleansing composition can be increased either by the careful selection of surfactants or by the inclusion of polymeric, organic or inorganic thickeners in the composition. Both methods are well known in the art. For example, U.S. Patents 4,912,823, 4,491,539, 4,556,510, 4,678,606 and 5,002,680 teach the use of polymeric thickeners to increase the viscosity of a liquid cleanser composition, while U.S. Patents 5,236,619, 5,132,037, 5,284,603, 5,296,158 and 5,158,699 disclose ways to formulate a stable viscous liquid composition using an appropriate combination of surfactants.

WO94/03151 relates to an aqueous detergent composition containing 5 to 50% by weight of a detergent which comprises 30 to 100% by weight of a fatty acylisethionate and 0 to 70% by weight of other detergent comprising 0.01 to 5% by weight of a cationic polymer and 0.5 to 15% by weight of silicone. The composition is an emulsion, with dispersed droplets of silicone oil.

The composition may include a cross-linked polyacrylate to increase the viscosity or enhance stability of the composition or a polysaccharide as a thickening agent.

US Patent No. 5,085,857 relates to an aqueous shampoo composition containing non-volatile silicone materials which condition the hair leaving it softer and more manageable.

The aqueous shampoo composition comprises 2 to 40% by weight of a surfactant, 0.01 to 3% by weight of a cationic guar gum conditioning polymer, and 0.01 to 10% by weight of an insoluble, non-volatile silicone. The non-volatile silicone is present as *emulsified* particles with an average particle size of less than 2µm.

The surface active agent can be selected from any known surfactant suitable for topical application to the human body.

One preferred anionic detergent is fatty acyl isethionate of formula

RCO₂CH₂CH₂SO₃M

where R is an alkyl or alkenyl group of 7 to 21 carbon atoms and M is a solubilizing cation such as sodium, potassium, ammonium or substituted ammonium. Preferably at least three quarters of the RCO groups have 12 to 18 carbon atoms and may be derived from coconut, palm or coconut/palm blends.

Another preferred anionic detergent is alkyl ether sulphate of formula:

RO(CH₂CH₂O)ₙSO₃M

where R is an alkyl group of 8 to 22 carbon atoms, n ranges from 0.5 to 10 especially 1.5 to 8, and M is a solubilizing cation as before.

Other possible anionic detergents include alkyl glyceryl ether sulphate, sulphosuccinates, taurates, sarcosinates, sulphoacetates, alkyl phosphate, alkyl phosphate esters and acyl lactates, alkyl glutamates uronic acid derivatives and mixtures thereof.

Sulphosuccinates may be monoalkyl sulphosuccinates having the formula:

R⁵O₂CCH₂CH(SO₃M)CO₂M;

and amido-MEA sulphosuccinates of the formula:

R⁵CONHCH₂CH₂O₂CCH₂CH(SO₃M)CO₂M;

wherein R⁵ ranges from C₈-C₂₀ alkyl, preferably C₁₂-C₁₆ alkyl and M is a solubilizing cation. Modified sulphosuccinates such as disodium citric acid polyethoxy lauryl ether sulphosuccinate (Rewopol SB CS 50 ex Witco) are also suitable.

Sarcosinates are generally indicated by the formula:

R⁵CON(CH₃)CH₂CO₂M,

wherein R⁵ ranges from C₈-C₂₀ alkyl, preferably C₁₂-C₁₆ alkyl.

Taurates are generally identified by the formula:

R⁵CONR⁶CH₂CH₂SO₃M,

wherein R⁵ ranges from C₈-C₂₀ alkyl, preferably C₁₂-C₁₅ alkyl, R⁶ ranges from C₁-C₄ alkyl, and M is a solubilizing cation.

Another useful surfactant is alkylethoxy carboxylate indicated by the following formula:

R⁵(OCH₂CH₂)ₙCOOM,

wherein R⁵ ranges from C₈-C₂₀ alkyl, preferably C₁₂-C₁₆ and M is a solubilized cation.

Harsh surfactants such as primary alkane sulphonate or alkyl benzene sulphonate will generally be avoided.

It should be understood that the term "anionic surfactant" covers fatty acid soaps.

Fatty acid soaps are typically alkali metal or alkanol ammonium salts of aliphatic alkane or alkene monocarboxylic acids. Sodium, potassium, mono-, di and tri-ethanol ammonium cations, or combinations thereof, are suitable for the purposes of the invention. The soaps are well known alkali metal salts of natural or synthetic aliphatic (alkanoic or alkenoic) acids having about 8 to 22 carbons, preferably 12 to about 18 carbons. They may be described as alkali metal carboxylates of acrylic hydrocarbons having about 12 to 22 carbons.

Examples of soap which may be used can be found in U.S. Patent No. 4,695,395 to Caswell et al. and U.S. Patent No. 4,260,507 (Barrett), both of which are incorporated herein by reference.

Suitable nonionic surface active agents include alkyl polysaccharides, aldobionamides (e.g., lactobionamides as described in US Patent 5389279), ethyleneglycol esters, glycerol monoethers, polyhydroxyamides (glucamide) including fatty acid amides as described in US Patent No. 5,312,934, hereby incorporated by reference), primary and secondary alcohol ethoxylates, especially the C₈₋₂₀ aliphatic alcohols ethoxylated with an average of from 1 to 20 moles of ethylene oxide per mole of alcohol.

The surface active agent is preferably present at a level of from 1 to 35% by wt., preferably 3 to 30% by wt.

It is also preferable that the composition includes from 0.5 to 15% by wt. of a cosurfactant with skin mildness benefits. Suitable materials are zwitterionic detergents which have an alkyl or alkenyl group of 7 to 18 carbon atoms and comply with an overall structural formula. 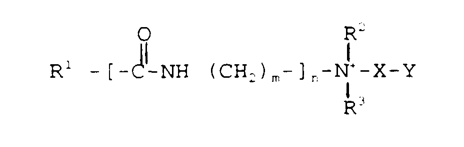 where
R¹ is alkyl or alkenyl of 7 to 18 carbon atoms,
R² and R³ are each independently alkyl, hydroxyalkyl or carboxyalkyl of 1 to 3 carbon atoms,
m is 2 to 4,
n is 0 or 1,
X is alkylene of 1 to 3 carbon atoms optionally substituted with hydroxyl, and Y is -CO₂- or -SO₃-.

Zwitterionic detergents within the above general formula include simple betaines of formula: 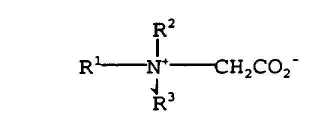 and amido betaines of formula: 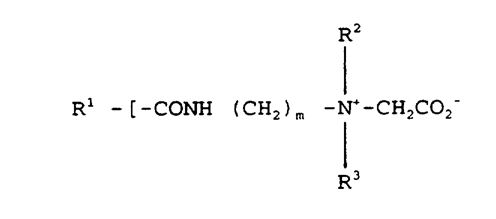 where m is 2 or 3.

In both formulae R¹, R² and R³ are as defined previously. R¹ may, in particular, be a mixture of C₁₂ and C₁₄ alkyl groups derived from coconut so that at least half, preferably at least three quarters, of the groups R¹ have 10 to 14 carbon atoms. R² and R³ are preferably methyl.

A further possibility is a sulphobetaine of formula: 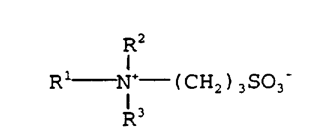 or 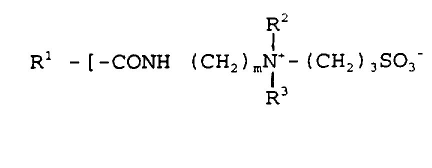 where m is 2 or 3, or variants of these in which -(CH₂)₃SO₃⁻ is replaced by 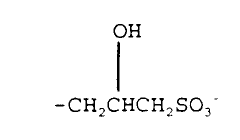 and R¹, R² and R³ in these formulae are as defined previously.

As noted, the hydrogel structure or composition comprises (ii) a hydrogel forming polymer system (containing at least two polymers) and (iii) a water insoluble benefit agent.

### (i) Hydrogel Forming Polymer System

Polymer systems useful for forming hydrogels with desirable properties comprise a first water-soluble polymer which insolubilizes when placed in an aqueous surfactant solution; and a second water-soluble property modifying polymer which ensures the hydrogel composition once formed has sufficient gel strength to retain the benefit agent but is weak enough so that it will disintegrate smoothly when the cleanser composition is applied to a substrate, e.g., skin.

Together the first and second polymers form a web or network which retains the benefit agent. Each of the polymers and the mechanism whereby the first polymer is insolubilized in the surfactant composition are discussed in greater detail below.

### (a) First Polymer

The first polymer is, in its broadest sense, defined as any water-soluble polymer that is made insoluble when placed in said aqueous surfactant solution (i). This insolubilization is accomplished by one of the following mechanisms:

### (1) Thermal gelation

Suitable polymers that exhibit this type of gelling behavior (i.e., can be made insoluble by thermal gelation) are those that are water-soluble at a temperature greater than about 40-50°C and which form a gel after a solution of the polymer is cooled to room temperature. Examples of such polymers include: i) gel forming polysaccharides; ii) gel forming proteins; and iii) thermally gelling synthetic polymers. Particularly preferred gel forming polysaccharides type are carrageenans, particularly carrageenan polymers such as those manufactured by FMC corporation and sold under the trade name Gelcarin GP911, and Gelcarin 379. Particularly preferred gel forming proteins are gelatins such as Gelatin G 9382 and G 2625 sold by Sigma Chemicals. Suitable thermally gelling synthetic polymers are poly (N-isopropylacrylamide) homo or copolymers or polyacrylate or methacrylate containing polymers that incorporate, as one of the monomer units, an acrylic or methacrylic ester of a long chain and, preferably, a linear alcohol. Examples of the latter class of polymers are those sold by Landec Labs Inc. under the trade name Inteliners.

### (2) Precipitation or coacervation:

Suitable polymers which exhibit this type of behavior are those polymers that are water-soluble at room temperature but can be made substantially insoluble by changing the physical or chemical properties of the aqueous solution such as, for example, by altering the pH or electrolyte concentration. One example of a particularly suitable pH sensitive polymer is Chitosan (polyglucosamine) or its various chemically modified variants. Particularly useful chitosans are those sold by Pronova Biopolymers under the trade name Seacure 343, and Seacure 443. These materials have a molecular weight ranging from 10,000 to 100,000. Chitosan is an especially useful network forming polymer for the hydrogel compositions of the present invention because it can be dissolved in an aqueous solution with a pH lower than 4.5 to form a uniform hydrogel precursor solution. Upon mixing this solution with a liquid cleanser which has a typical pH in the range of 5.0 to 7.0, an insoluble hydrogel network is readily formed. Another class of polymers that form suitable hydrogel networks by precipitation/coacervation are electrolyte sensitive polymers. These polymer will precipitate to form insoluble networks by adding the polymer solution to an aqueous surfactant solution containing high levels of salt or ionic surfactants. Examples of such polymers include polyvinyl alcohol having a molecular weight greater than 13,000 Daltons and a degree of hydrolysis in the range of 78% to 100%; and hydroxyethylcellulose such as those sold by Aqualon Corp. under the trade name Natrosol.

It should be understood that the same polymer may be in either class (2)(a) (i.e., water-soluble, but insolubilized upon contact with said aqueous surfactant solution); or class (2)(b) (i.e., soluble in both water and aqueous surfactant solutions) depending on what else is present in said aqueous surfactant solution. Thus, a polymer sensitive to electrolyte concentration might be a class (2)(b) polymer in a composition without electrolyte, but become a class (2) (a) polymer in an aqueous surfactant solution containing sufficient electrolyte to insolubilize the polymer. The same is true if certain polymers become insoluble in the presence of certain cross-linkers. If the cross-linker is not present in the aqueous surfactant solution, the polymer may be a (2)(b) polymer, but, if present, the polymer becomes a (2)(a) polymer.

### (3) Cross-linking:

Suitable water-soluble polymers exhibiting this type of behavior are those that can form water-insoluble complexes with a water-soluble cross-linker. The insoluble polymer network can be formed by reacting an aqueous solution of the polymer with the cross-linker before or after adding it into the aqueous surfactant solution. Examples of readily cross-linkable polymers include κ-Carrageenans which can be cross-linked with potassium ions; alginates which can be cross-linked with calcium ions and polyvinyl alcohols which can be cross-linked with borax ions.

### (b) Second (Property Modifying) Polymer

The first polymers described above provide the backbone network without which the hydrogel composition cannot exist. However, they alone are usually not sufficiently surface active in the hydrogel precursor solution to effectively stabilize emulsions of or dispersions of the water-insoluble cosmetic benefit agents. A second more surface active water-soluble polymer was found necessary to form dispersions having the required stability.

A second function of the second or property modifying polymer is to assist in the control of gel strength. As stated above, it is desirable that the hydrogel composition is sufficiently strong such that the dispersed hydrogel domains/particles remain discrete in order to effectively trap the cosmetic benefit agent within their structure, yet be sufficiently flexible (non-rigid) that the hydrogel domains/particles rub smoothly into the skin during use. This balance can be achieved by optimizing the gel strength of the hydrogel composition.

It has been found that certain second water-soluble polymers when incorporated into the hydrogel precursor solution along with the first polymer can effectively modify the gel strength of the hydrogel composition to achieve this balance. The hydrogel strength and the in-use skin feel properties can thus be manipulated easily by controlling the amounts of the network forming polymer (first polymer) and of the second property modifying polymer in the hydrogel precursor solution.

A variety of hydrophilic anionic, cationic, amphoteric, and nonionic water-soluble polymers can be utilized for this purpose. The second polymer can be soluble in the aqueous surfactant solutions (in contrast to the first polymers which are not) and have a molecular weight greater than 5,000 Daltons, preferably greater than 10,000 Daltons, and most preferably greater than 50,000 Daltons.

Examples of preferred second water-soluble polymers that have been found useful as property modifying polymers include: i) carboxylic acid containing acrylic polymers such as alkali soluble polyacrylic latexes sold under the trade name of Acrysol or Aculyn by Rohm & Haas; and cross-linked polyacrylic acids and copolymers sold by B. F. Goodrich under the trade name Carbopol; ii) nonionic polymers such as polyvinyl alcohol from Air Products sold under the trade name Airvol; polyvinyl pyrrolidone from ISP Technologies Inc; modified corn starch such as those sold under the trade name of Capsule or Purity Gum Bee by National Starch & Chemicals; and hydroxyethylcellulose sold by Aqualon under the trade name of Natrosol; and iii) cationic polymers such as modified polysaccharides including cationic guars available from Rhone Poulenc under the trade names Jaguar C13S, and Jaguar C14S; cationic modified cellulose such as Ucare Polymer JR 30 or JR 40 from Amerchol; synthetic cationic polymers such as polydimethyldiallyammonium chloride homo- or copolymers sold under the trade name Merquat 100, Merquat 550 sold by Calgon, and vinyl pyrrolidone/dimethylamino ethyl methacrylate copolymer sold under the trade name of Gafquat 755 from GAF Chemical.

The second component of the "hydrogel" composition is the cosmetic benefit agent (component (iii) above). This is described in greater detail below.

### (iii) Cosmetic Benefit Agent

Cosmetic benefit agents in the context of the present invention are materials that have the potential to provide a positive and often longer term effect on the substrate being treated therewith, e.g., to the skin, hair or teeth. In the case of skin, suitable cosmetic benefit agents are water insoluble materials that can protect, moisturize or condition the skin after being deposited from the liquid cleanser composition.

Preferred cosmetic benefit agents include:
a) silicone oils, gums and modifications thereof such as linear and cyclic polydimethylsiloxanes; amino, alkyl alkylaryl and aryl silicone oils;
b) fats and oils including natural fats and oils such as jojoba, soybean, sunflower, rice bran, avocado, almond, olive, sesame, persic, castor, coconut, mink oils; cacao fat, beef tallow, lard; hardened oils obtained by hydrogenating the aforementioned oils; and synthetic mono, di and triglycerides such as myristic acid glyceride and 2-ethylhexanoic acid glyceride;
c) waxes such as carnauba, beeswax, lanolin and derivatives thereof;
d) hydrophobic plant extracts;
e) hydrocarbons such as liquid paraffins, petrolatum, microcrystalline wax, ceresin, squalene, squalane, pristan and mineral oil;
f) higher fatty acids such as those having 8 to 24 carbons atoms, for example, lauric, myristic, palmitic, stearic, behenic, oleic, linoleic, linolenic, lanolic, isostearic and poly unsaturated fatty acids (PUFA) acids;
g) higher alcohols such as those having 8 to 24 carbon atoms, for example, lauryl, cetyl, steryl, oleyl, behenyl, cholesterol and 2-hexadecanol" alcohol;
h) esters such as cetyl octanoate, myristyl lactate, cetyl lactate, isopropyl myristate, myristyl myristate, isopropyl palmitate, isopropyl adipate, butyl stearate, decyl oleate, cholesterol isostearate, glycerol monostearate, glycerol distearate, glycerol tristearate, alkyl lactate, alkyl citrate and alkyl tartrate; sucrose ester sorbitol ester, and the like;
i) essential oils such as fish oils, mentha, jasmine, camphor, white cedar, bitter orange peel, ryu, turpentine, cinnamon, bergamot, citrus unshiu, calamus, pine, lavender, bay, clove, hiba, eucalyptus, lemon, starflower, thyme, peppermint, rose, sage, menthol, cineole, eugenol, citral, citronelle, borneol, linalool, geraniol, evening primrose, camphor, thymol, spirantol, pinene, limonene and terpenoid oils;
j) lipids and lipid like substance such as cholesterol, cholesterol ester, ceramides, sucrose esters and pseudoceramides as described in European Patent Specification No. 556 957;
k) vitamins such as vitamin A and E, and vitamin alkyl esters, including vitamin C alkyl esters;
l) sunscreens such as octyl methoxyl cinnamate (Parsol MCX) and butyl methoxy benzoylmethane (Parsol 1789);
m) Phospholipids such as lecithins;
n) antimicrobial such as 2-hydroxy-4,2',4'-trichlorodiphenylether (DP300) and 3,4,4'-trichlorocarbanilide (TCC); and mixtures of any of the foregoing components.
o) derivatives of alpha hydroxy acids such as materials of formula 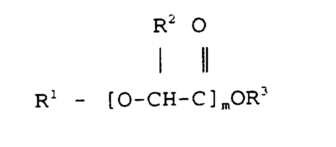 wherein
   R¹ is CₚH_{q}NᵣOₛ, where p is 0-20, q is 1-41, r is 0-3, and
   s is 0-3;
   R² is CₜHᵤ where t is 0-20 and u is 1-41;
   R³ is CᵥH_{w}NₓO_{y} where v is 0-20, w is 1-41, x is 0-3 and y is 0-3 or a metallic, ammonium or alkanolammonium anion; and m is 1-10; and
   mixtures of any of the foregoing components.

The cosmetic benefit agent can be incorporated into the hydrogel precursor solution (the solution that contains the first and second polymers). This can be accomplished simply by mixing the benefit agent with the hydrogel precursor solution or by preemulsifying the benefit agent in an aqueous solution which is then mixed with the hydrogel precursor solution. The benefit agent is present in an amount of 5 to 65, preferably 20 to 50 % by wt. based on the hydrogel composition. Depending on the mixing conditions, polymer composition (i.e., composition of polymers forming the hydrogel) and concentration, the size of the benefit agent particle can vary from 0.2 up to 200 µm. For better stability and efficient deposition, the particle size of benefit agent is preferably in the range of 5 to 150 µm.

Compositions of the invention may be formulated as products for washing the skin, e.g., bath or shower gels, hand washing compositions, facial washing liquids; pre- and post-shaving products; rinse off, wipe off and leave on skin care products.

The compositions of the invention will generally be pourable liquids or semi liquids e.g., pastes and will preferably have a viscosity in the range 1500 to 100,000 mPas measured at a shear rate of 10s⁻¹ at 25°C in a Haake Rotoviscometer RV20.

Other typical components of the compositions include opacifiers, preferably 0.2 to 2.0% by wt.; preservatives, preferably 0.2 to 2.0% by wt. and perfumes, preferably 0.5 to 2.0% by wt.

The invention will now be illustrated by the following nonlimiting examples.

All percentages mentioned are intended to be percentages by weight unless indicated otherwise.

### EXAMPLES

### Example 1. Preparation of a hydrogel composition containing silicone oil by a direct mixing process.

There are a number of ways that a polymeric hydrogel can be dispersed into an aqueous surfactant solution in such a way that the hydrogel forms discrete domains. All of the techniques we have found useful take advantage of *rapid* gelation or cross-linking that occurs for certain types of water soluble polymers when their solution environment changes, e.g., a change in pH, ionic strength, temperature, type of ion, presence of cross-linker, etc.

This example illustrates how a hydrogel composition can be prepared by directly mixing an appropriate hydrogel forming polymer solution (called the *hydrogel precursor solution*) with an aqueous surfactant solution using a conventional mechanical stirrer. In this case, the polymer system is chosen such that the change in pH that arises when the hydrogel precursor solution is mixed with the surfactant solution induces a phase changes (gelation). Gelation is sufficiently rapid that the added precursor hydrogel solution (containing (1) first polymer which insolubilizes when added to aqueous surfactant solution; (2) second property modifying polymer and (3) benefit agent), which itself is water continuous, does not first dissolve in the surfactant solution but rather forms a discrete dispersed aqueous phase.

### Preparation of hydrogel precursor solution

A 40% emulsion of silicone oil (60,000 centistokes, ex Dow Corning) was formed by mixing 40 parts of the silicone oil to 60 parts an of aqueous polymer solution which contained 1% by wt. of Chitosan (first polymer) (Sea Cure 340 ex. Protan), 0.8% by wt. of acetic acid and 3% by wt. of hydroxyl ethylcellulose (second polymer) (Natrosol 250 MR ex. Aqualon) at 400 rpm for 30 minutes using an overhead stirrer. The emulsion so prepared contained droplets of silicone oil having a broad size distribution ranging from about 4 µm to 50 µm dispersed in the polymer solution.

### Preparation of hydrogel dispersion by direct mixing

12.5 parts of the hydrogel precursor solution containing emulsified silicone oil described above was added to 87.5 parts of an aqueous surfactant solution (composition Table 1). Mixing was accomplished with an overhead stirrer (Model RW20DZM ex Tekmar). Two dispersions were prepared that had identical chemical composition but differed in the stirring speed used to form them. In Example 1A, the dispersion was mixed at 80 RPM for 25 minutes while in Example 1B the mixing speed was at 200 RPM. Both examples contained irregular shaped hydrogel domains that themselves contained emulsified silicone oil droplets entrapped within the gel matrix.

The compositions of Examples 1A and 1B are given in Table 2 along with some of their physical properties.

**Table 1.**

| **Liquid Cleanser Composition** | |
|---|---|
| **Ingredient** | **Wt%** |
| Cocamidopropyl Betaine | 5.6 % |
| Sodium Cocoyl Isethionate | 3.5 % |
| Sodium Laureth Sulfate | 1.4% |
| Glydant XL | 0.14% |
| Ammonium Sulfate | 0.09% |
| Jaguar C13S | 0.9% |
| Perfume | 0.42% |
| Deionized Water | 87.95% |

**Table 2.**

| **Composition and properties of hydrogel dispersions formed by direct mixing.** | | | |
|---|---|---|---|
| | Example 1A | Example 1B | Example 2 |
| Ingredient | Wt% | Wt% | Wt% |
| Silicone oil | 40% | 40% | 40% |
| Chitosan | 0.6 % | 0.6 % | 0.6 % |
| Hydroxyethylcellulose | 1.8% | 1.8% | - |
| Jaguar C13S | - | - | 1.8% |
| water | to 100 | to 100 | to 100 |
| Silicone Oil drop size | 4-50 µm | 4-50 µm | 1-25 µm |
| Hydrogel domain size | 255 nm | 227 nm | 157 nm |

### Example 2. Further illustration of hydrogel composition containing silicone oil prepared by direct mixing

A hydrogel precursor solution similar to the one described in Example 1 but having a different polymer composition was prepared as follows. 40 parts of 60,000 centipoise silicone oil was emulsified in 60 parts of aqueous polymer gel solution which contained 1% by wt. of Chitosan (first polymer) (Sea Cure 340 ex. Protan), 0.8% by wt. of acetic acid and 3% by wt. of a cationic guar polymer (second polymer) (Jaguar C13S ex. Rhone-Poulenc) at 400 rpm for 30 minutes using an overhead stirrer.

12.5 parts of this hydrogel precursor solution was then added to 87.5 parts of a surfactant solution (formulation given in Table 1) and mixed at 80 rpm for 25 minutes. This process lead to a dispersion that contained irregular shaped particles of hydrogel in which silicone oil droplets were entrapped in the gel matrix. The composition and properties of this dispersion are given in Table 2.

### Example 3. Hydrogel composition containing silicone oil prepared by extrusion

This example demonstrates that hydrogel dispersions can also be prepared by a coextrusion process. Specifically the example illustrates compositions prepared by coextruding a hydrogel precursor solution (containing in this case dispersed silicone oil) with an aqueous surfactant solution by means of a two fluid nozzle. This process produces hydrogel compositions dispersed in the form of noodle shape particles suspended in the final liquid cleanser composition. The noodle shape hydrogel dispersion gives a unique appearance to the liquid cleanser. Alternatively, the dispersion can be processed further by any number of shear techniques to chop or subdivide the noodles into a desired size range. The process is illustrated for a silicone oil containing hydrogel composition, but can be used in conjunction with a great variety of materials as will be shown in the subsequent examples.

### Preparation of hydrogel precursor solution with dispersed silicone oil

Silicone oil was mixed with an aqueous solution of a neutralized carboxyl acid containing an acrylic copolymer (second polymer) (Aculyn-33, Rohm & Haas) in an aqueous solution with a pH in the range of 7 to 7.5 by mechanical stirring. In this case Aculyn-33 was the property modifying polymer. The oil droplet size, which varied from 5 to 150 µm in diameter, was controlled by the concentration of Aculyn-33 and the stirring speed. Higher concentration and faster stirring led to smaller oil droplets. An aqueous κ-carrageenan (Gelcarin GP-911, FMC) solution, the first polymer (network forming polymer), was added into the mixture with gentle stirring (< 100 rpm) at 45-50°C. In this example two hydrogel precursor dispersions were prepared that differed in the size of the silicone oil droplets. In one dispersion, the silicone oil droplet size was in the range 2-30 µm while in the second dispersion the droplet size was in the range 40-100 µm.

### Preparation of hydrogel dispersion by coextrusion

The warm (45-50°C) precursor solution described above was fed into the central orifice of a commercially available nozzle (Series No. 1/4 XA SR450 A ex Bete Fog Nozzle Inc.) by means of a high pressure syringe pump (Syringe pump model 40 ex Harvard Apparatus). Simultaneously, a cool (room temperature ~ 22°C) shower gel formulation (composition given in Table 3) was injected to the outer orifice of the nozzle to form the final product which contained "noodle-shaped" hydrogel composition particles. A high pressure syringe pump was also used to transfer the shower gel composition. The hydrogel and silicone contents in the final dispersion were controlled by varying the ratio of the flow rates of the hydrogel precursor and the surfactant compositions. This ratio was adjusted to ensure that the final product contained 5% by wt. silicone oil.

The characteristics of the two hydrogel dispersions, Example 3A and Example 3B, are given in Table 4. They consisted of noodle shaped particles of approximately 1000 µm in diameter suspended in the shower gel composition of Table 3.

**Table 3.**

| **Base shower gel formulation** | |
|---|---|
| Ingredient | % by wt. |
| Sodium Lauryl Ether(3) Sulfate | 4 |
| Cocoamido Propyl Betaine | 1 |
| Dodecyl polyglycoside | 5 |
| Perfume | 1 |
| Sorbic Acid | 0.37 |
| Sodium Citrate 2H₂O | 0.49 |
| Sodium Hydroxide¹ | 1 |
| Carbopol ETD2020² | 0.9 |
| Antil 141³ | 0.97 |
| H₂O | to 100 |

| | |
|---|---|
| Footnote 1: Adjust pH to 5.24 ± 0.1 with NaOH | |
| Footnote 2: Cross-linked polyacrylic acid | |
| Footnote 3: Polyethylene glycol propylene glycol oleate | |

**Table 4.**

| **Characteristics of "Noodle Shaped" Hydrogel Particles formed by coextrusion (Example 3).** | | | | | | |
|---|---|---|---|---|---|---|
| Example | Hydrogel Phase | | | Oil Phase Silicone Oil (60,000 cs) | | Diameter of Noodle's Cross-section (µm) |
| | Polymer | | Water | % by wt. | Droplet Size (µm) | |
| | Type | % by wt. | % by wt. | | | |
| 3A | Carrageenan/Aculyn | 1.2/0.4 | 38.4 | 60.0 | 40-100 | 1000 |
| 3B | Carrageenan/Aculyn | 1.2/0.4 | 38.4 | 60.0 | 2-30 | 1000 |

Aculyn is a neutralized carboxyl acid containing acrylic copolymer.

### Example 4. Effect of Polymer Composition on hydrogel properties.

This method illustrates how the rheological properties of the hydrogel which affect their in-use properties can be manipulated by the selection and concentrations of the network-forming (first) and property modifying (second) polymers.

A series of model hydrogel particles were prepared by a cross-linking method using carrageenan as the network polymer and potassium ions as the cross-linking agent. The dispersions were made by a variant of the extrusion process described in Example 3 using an aqueous solution of KCl as the model cleansing composition. The hydrogel dispersions differed only in the concentration of carrageenan (first polymer) used in the hydrogel precursor solution which ranged from 1 to 6% by wt. Approximately spherical particles were formed all having a diameter of about 3000 µm.

An Instron (Model 1122) was found to be a suitable instrument to differentiate the strength of various gel compositions described above. The measurement procedure was as follows. A hydrogel particle was carefully removed from the dispersion with a spatula and excess liquid was removed by placing them on a kimwipe. The approximate diameter of the hydrogels was measured with calipers. The hydrogel was then transferred to a metal platform base of the Instron and a 10 Newton load was lowered until it came in contact with the hydrogel particle. The Instron was then turned on and the force was measured as a function of distance using a chart recorder. The chart recorder was set at a crosshead speed of 1 mm/min and a chart speed of 50 mm/min which allowed for a 2 Newton full scale load reading. The force was recorded at 0.50 mm, 1.00 mm, and 1.50 mm. The elastic modulus of the sample was calculated by dividing the force by the section area of the hydrogel and the strain. It should be noted that the particles remained hydrated during the course of the measurement.

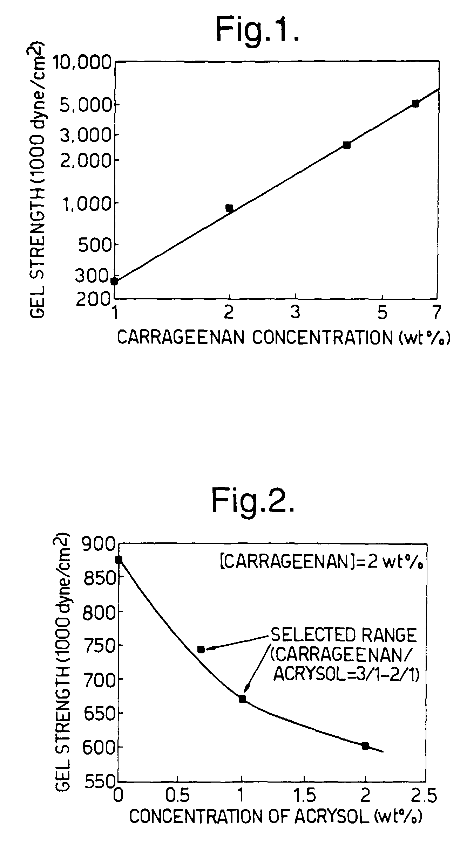

The gel strength measured by the above procedure for the series of model hydrogel particles are given in Figure 1. These results indicated that the gel strength increased significantly with increasing carrageenan concentration. When the polymer concentration was higher than 2% by wt., the gel felt quite hard to the touch, and did not rub smoothly into the skin. Although such particles might trap benefit agents and remain stable during storage, they would not be optimum for cleansing applications because the delivery of the benefit agent would not be uniform, and the composition would feel gritty. These results also indicate that if it is desired to decrease the gel strength of the hydrogel particles this could only be achieved by decreasing the concentration of polymer within the gel. This would greatly limit the flexibility of the technology. As will be shown below, this limitation can be overcome by using a second polymer to modify the properties of the hydrogel, e.g., its gel strength.

Another set of model hydrogel particles were prepared by the same procedures used above. Each hydrogel precursor solution contained 2% by wt. carrageenan and from 0 to 2% by wt. of an acrylic copolymer, Acrysol ASE-60 (Rohm & Haas). This is the second property modifying polymer. The shape and size of the hydrated particles were all very similar and also similar to the all carrageenan hydrogels described previously. The influence of added Acrysol ASE-60 on the gel strength of hydrogel particles formed by cross-linking with K⁺ is shown in Figure 2. The results indicate that the gel strength decreased significantly with increasing Acrysol concentration even though the total concentration of polymer increased. Hydrogels containing carrageenan and Acrysol in a weight ratio ranging from 3:1 to 2:1 (arrows in Figure 2) were capable of effectively trapping benefit agents such as emulsified emollient oil e.g., silicone oil, and yet rubbed smoothly into the skin without noticeable grit.

The above example is set forth for illustrative purposes to show how the gel strength can be tuned by selection of a polymer system comprising an network-forming and property modifying polymer. Care must be taken not to attach too much significance to the exact values of gel strength quoted above. We have found that the quantitative effect of the polymer composition in the hydrogel precursor solution on the strength of a hydrogel particle depends significantly on the nature, particle size, and concentration of a benefit agent that may be dispersed in the precursor solution. Although the trends are the same, the exact values may change.

Although complex, one skilled in the art can utilize a combination of polymers broadly falling in the definition of network-forming and property modifying agents to achieve the proper balance of gel strength for each situation.

### Example 5. Deposition of silicone oil on skin ex-vivo.

The potential of various cleansing compositions to deposit silicone oil onto the skin during the washing process was determined using an ex-vivo model based on pig skin. The procedure is described below.

A 0.5 ml aliquot of the test composition was applied to 2" by 2" square strips of excised pig skin that had first been prewetted with tap water at 37°C. The composition was lathered for 10 seconds and then rinsed for 10 seconds under warm running tap water. The skin was then wiped once with a paper towel to remove excess water and allowed to dry for two minutes. A strip of adhesive tape was then pressed onto the skin for 30 seconds under a load of 10 g/cm². The adhesive tape employed was J-Lar Superclear (TM) tape having a width of 3 cm. In this test procedure, the silicone, which had deposited on the skin, will subsequently be transferred to the tape along with some of the outer skin layers.

The amounts of silicone and skin adhering to the tape were determined by means of X-ray fluorescence spectroscopy. The tape strips were placed in an X-ray fluorescence spectrometer with the adhesive side facing the beam of this machine. A mask is applied over the tape to define a standardized area in the middle of the tape which was exposed to the X-ray beam. The sample chamber of the machine was placed under vacuum before making measurements and the spectrometer was then used to measure the quantities of silicone and sulphur. The sulphur was representative of the amount of skin which had transferred to the tape.

### The amount of silicone and sulphur observed with a clean piece of adhesive tape were subtracted from the experimental measurements. The experimental measurements for successive pieces of tape were added together and the cumulative totals of silicone and sulphur were expressed as a ratio of silicone to sulphur per unit area of skin. A higher Si/S ratio corresponds to a higher deposition level. A total of 10 tape strips were employed for each measurements.

The amount of silicone deposited on excised pig skin determined as described above for the compositions described in Examples 2-3 are listed in Table 5. It is clear from these results that the hydrogel compositions provided significant deposition of silicone oil to the skin in these tests.

**Table 5.**

| **Deposition of Silicone from Shower Gel** | |
|---|---|
| Example No. | Silicone Deposition (Silicon/Sulphur Ratio) |
| Example 2 | 1.38 |
| Example 3A | 28.0 |
| Example 3B | 7.1 |
| Control 1 (Note 1) | 0.3 |

| | |
|---|---|
| Note 1. The control in this example is the shower gel composition given in Table 3 which did not contain any silicone. | |

### Example 6. Hydrogel dispersions made with different polymers.

This example illustrates that hydrogel dispersions can be prepared with a variety of polymers provided they possess the required hydrogel forming capabilities and gel strengths. In the composition described below, a cationic polymer Chitosan (first polymer) (Seacure 343 ex. Pronova Biopolymer) was used as the network forming polymer and a cationic modified guar (Jaguar C-13S, Calgon) and a synthetic cationic polymer polydimethyl- dialkylammonium chloride (Merquat 100, Calgon) were used as the second or property modifying polymers. The hydrogel precursor solution was prepared by mixing 35 parts of 60,000 cps silicone oil with 65 parts of polymer solution containing 1.5% by wt. Jaguar C13S, 0.75% by wt. Chitosan, 0.10% by wt. Merquat 100, 0.35% acetic acid and 0.05% by wt. cocoamido propylbetaine at 300 rpm for 15 minutes using an overhead stirrer. The resulting emulsion contained silicone oil droplets with size ranging from 2 µm to 60 um dispersed in the polymer solution.

The extrusion process described in Example 3 was used to prepare 14.3% by wt. dispersions (5% by wt. silicone oil based on the total composition) of the hydrogels. The surfactant system was the same as the one used in Example 3 (Table 3) except 0.3 instead of 0.97% Antil 141 was used in the formula. The hydrogel dispersion comprised noodle shaped particles dispersions that contained dispersed silicone oil having an average droplet size in the range of 2-60 µm.

The deposition of silicone oil from the composition of Example 6 is given in Table 6. The deposition tests were carried out according to the method described in Example 5. The dispersion exhibited a similar deposition to other hydrogels having similar silicone droplet size suggesting that the nature and characteristics of the dispersions may be more important in determining the level of deposition than the exact nature of the polymer employed.

**Table 6.**

| **Deposition of silicone oil onto pig skin from cleanser compositions containing dispersed hydrogels.** | |
|---|---|
| **Sample No.** | **Si:S** |
| Example 6 | 6.78 |
| Control (same control as in Table 5) | 0.3 |

### Example 7. Illustration of the enhanced deposition produced by hydrogels.

One of the benefits of using the hydrogel dispersions of this invention is the enhanced deposition of benefit agents incorporated within their structure relative to the benefit agents used by itself. This example illustrates the effect for silicone oil. In particular, deposition of silicone oil from various skin cleansing compositions were compared in the deposition test described in Example 5. A set of compositions was prepared by directly emulsifying 60,000 cps silicone into the cleansing composition of Table 3 to make the droplet size of silicone oil about the same as those contained in the hydrogel dispersions of Examples 3A, 3B and 6.

The deposition results of hydrogel dispersions vs. silicone emulsion alone are given in Table 7 and demonstrate that a substantial enhancement in deposition can be achieved by incorporating the agent within a dispersed hydrogel domain.

**Table 7.**

| **Enhancement of deposition of silicone oil from hydrogel**^{**(a)**}**.** | | | |
|---|---|---|---|
| **Sample No.** | **Silicone oil Droplet Size** | **Deposition Level**^{**(b)**} | |
| | | **Si/S Ratio** | **Si/S Ratio** |
| | | **Silicone in Hydrogel** | **Silicone Emulsions Alone**^{**(c)**} |
| Example 3B | 2-30 µm | 7.1 | 1.2 |
| Example 3A | 40-100 µm | 28.0 | 8.3 |
| Example 6 | 2-60 µm | 6.78 | 2.0 |

| | | | |
|---|---|---|---|
| Note (a). All compositions employed the surfactant base listed in Table 3. The concentration of silicone oil in the final composition was 5 wt% in all cases. | | | |
| Note (b). Deposition was measured by the ex-vivo method described in Example 5. | | | |
| Note (c). The same silicone oil were used in the preparation of the skin cleansing compositions by directly emulsifying the silicone oil into the liquid cleanser. | | | |

### Example 8. Preparation of hydrogel dispersion containing hydrocarbon oils

A wide variety of ingredients can be incorporated into hydrogel dispersions. Previous examples have illustrated several general features of these dispersions using silicone oils. Another class of useful benefit agents are hydrocarbon oils. Some of the benefit agents of this class of materials are moisturizing agents such as petrolatum, emollients such as isopropyl palmitate and sunscreens such as Parsol MCX (2-Ethylhexyl-P-Methoxy Cinnamate). These examples illustrates the use of hydrogels to deliver such hydrocarbon oils.

### Preparation of hydrogel precursor solution

40% emulsions of different hydrocarbon oils i.e. 100% petrolatum, a mixture of 50% isopropyl palmitate and 50% petrolatum, and a mixture of 50% Parsol MCX and 50% of petrolatum were prepared by mixing 40 parts of the hydrocarbon oil to 60 parts of aqueous polymer solution which contained 0.5% by wt. Chitosan (first polymer) (Seacure 343 ex Pronova Biopolymer), 1.9% by wt. Jaguar C13S (second polymer), 0.4% by wt. cocoamido propylbetaine and 0.25% by wt. acetic acid at 300 rpm for 20 minutes using an overhead stirrer. The mixing was carried out at room temperature except for the 100% petrolatum sample. The 100% petrolatum emulsion was prepared by melting the petrolatum at 60°C which was then added to the room temperature polymer solution and mixed at room temperature.

### Preparation of hydrogel dispersion by coextrusion

The extrusion process described in Example 3 was used to prepared 12.5% by wt. dispersion of the hydrogels containing 100% petrolatum, 50% isopropyl palmitate/50% petrolatum, and 50% Parsol MCX/50% petrolatum respectively for examples 8A, 8B and 8C. The surfactant system was the same as the one used in Example 3 (Table 3) except no Antil 141 was used in the formula. All these samples comprised noodle shaped hydrogel dispersions with diameter about 1000 nm.

### Examples 9. Preparation of hydrogel dispersion containing solid fatty acid

Previous examples have illustrated a wide variety of hydrophobic oils can be incorporated into the hydrogel dispersions of the instant invention. Another class of useful materials are dispersed hydrophobic solids or waxy particles. Some of the benefit agents within this class of materials are moisturizing agents such as fatty acids, emollients, certain solid sunscreens such as p-hydroxybenzoic acid, antimicrobial agents such as Triclosan, and anti-acne agents such as salicylic acid.

This example illustrates the use of hydrogels to deliver such solid or waxy hydrophobic solids using a mixture of solid fatty acids as the specific example.

### Preparation of hydrogel precursor solution.

22 grams of a modified corn starch (Capsul ex. National Starch & Chemical), 4.4 grams of Carrageenan (Gelcarin GP911 ex. FMC) and 0.55 gram of sodium lauryl sulfate (ex BDH) were dissolved in 83.05 grams of deionized water at 80°C. 210 grams of a molten fatty acid mixture containing 50% by wt. of palmitic acid and 48% by wt. of stearic acid was added to the above polymer solution and emulsified to form a fatty acid emulsion at 60°C. The fatty acid emulsion was then transferred to a Hobart Kitchen Aid mixer and mixed whilst cooling to form a viscous dough-like fatty acid emulsion.

The emulsion comprised fatty acid particles with a size in the range of 1 to 40 µm in diameter.

The above composition was then mixed with an equal amount of polymer solution containing 4% of Merquat 100 (ex. Calgon), 0.5% of Chitosan (Sea Cure 340 ex. Protan) and 0.4% of acetic acid to form a hydrogel precursor solution containing dispersed fatty acid.

### Preparation of hydrogel by extrusion

14 parts of the hydrogel precursor solution was then coextruded with 86 parts of the liquid cleanser of Table 3 using the method described in Example 3 to form hydrogel dispersion. This dispersion consisted of noodle shaped gel particles uniformly distributed in the cleanser composition.

### Example 10. Further illustration of hydrogel dispersion containing solid fatty acid

This example is very similar to the previous one except that a different gel forming polymer was employed.

### Preparation of hydrogel precursor solution.

22 grams of a modified corn starch (Capsul ex. National Starch & Chemical), 4.4 grams of Carrageenan (Gelcarin GP911 ex. FMC) and 0.55 gram of sodium lauryl sulfate (ex BDH) were dissolved in 83.05 grams of deionized water at 80°C. 210 grams of a molten fatty acid mixture containing 50% by wt. of palmitic acid and 48% by wt. of stearic acid was added to the above polymer solution and emulsified to form a fatty acid emulsion at 60°C. The fatty acid emulsion was then transferred to a Hobart Kitchen Aid mixer and mixed while cooling to form a viscous dough-like fatty acid emulsion.

The emulsion comprised fatty acid particles with a size in the range of 1 to 40 um in diameter.

The above composition was then mixed with an equal amount of a polymer solution containing 10% by wt. of fully hydrolyzed polyvinyl alcohol solution (Airvol 350 ex. Air Product).

### Preparation of hydrogel by extrusion

14 parts of the hydrogel precursor solution was then coextruded with 86 parts of the liquid cleanser of Table 3 using the method described in Example 3 to form hydrogel dispersion. This dispersion, consisted of noodle shaped gel particles uniformly distributed in the surfactant composition.

### Example 11. Deposition of Fatty Acid from Liquid Cleanser

The deposition of fatty acid onto pig skin was measured by a gas chromatograph method. A piece of pig skin (approximately 5 cm by 5 cm) was first washed with about 0.35 grams of a liquid cleanser for one minute. The treated pig skin was then rinsed with water for 50 seconds. It skin was dried once using a paper towel, air dried for two minutes and then extracted with 10 grams of heptane for 30 minutes. Aliquats of the heptane solution were injected into a GC to determine the amount of fatty acid deposited onto the skin. This was then repeated using a control sample comprised 7 parts water, 7 parts of the fatty acid emulsion used to prepare the dispersions in Examples 9 and 10 and 86 parts of the surfactant composition listed in Table 3 (also used to prepare Examples 10 and 11). The amount of fatty acid deposited from Examples 9 and 10, are compared with the control in Table 8. The result demon-strate that significant deposition of fatty acid occurs from these hydrogel dispersions. Furthermore, as with silicone oil, incorporating the fatty acid in the hydrogel formulation significantly increases its deposition (compare Examples 9 and 10 with control).

**Table 8.**

| **Deposition of fatty acid from hydrogel dispersions under conditions typical of skin cleansing.** | |
|---|---|
| Sample No.^{(a)} | Deposition of Fatty Acid µg /cm² |
| Example 9 | 5.10 |
| Example 10 | 4.2 |
| Control^{(b)} | <0.5 |

| | |
|---|---|
| Note (a). All samples contained 4% by wt. fatty acid and the same fatty acid emulsion was used to prepare all the samples. | |
| Note (b). The control was prepared by mixing 7 parts of the fatty acid emulsion used to prepare Examples 9 and 10 with 7 parts of water. This was then mixed with 86 parts of the surfactant composition of Table 3. | |

### Example 12. Preparation of hydrogel composition containing lipid

A molten lipid solution was prepared by heating 30 parts glycerol, 15 parts Cholesterol (Cholesterol USP ex. Croda Chemicals LTD), 7.5 parts sugar ester (Ryoto S270 ex. Mitsubishi-Kasei Foods Corp.) and 7.5 parts stearic acid (ex. Unichema International) on a 150°C hot plate. The clear molten lipid solution was then cooled to about 98°C and mixed with 10 parts of neutralized Aculyn 33 (ex. Rohm & Haas, 2% by wt., pH: 7.5-8.0) solution and 30 parts of Carrageenan Gelcarin GP911 solution (2% by wt.) to form a hydrogel dispersion precursor containing cholesterol.

The extrusion process described in Example 3 was used to prepare 17% by wt. dispersion (2.55% by wt. Cholesterol based on the total composition) of the hydrogel. The same surfactant system as Example 3 (Table 3) was used for the preparation. Deposition of cholesterol from this liquid cleanser composition was determined by the method described in Example 13. A comparative example containing 2.5% by wt. cholesterol uniformly dispersed in a liquid cleanser was prepared for comparison. The composition and the preparation process of this comparative example is given in Table 9 below.

**Table 9**

| Ingredient | % wt. |
|---|---|
| Cholesterol | 2.50 |
| Stearic Acid | 1.25 |
| Sucrose Ester | 1.25 |
| Glycerol | 5.00 |
| Genapol ZRO (SLES) Sodium Lauryl Ether Sulfate | 16.0 |
| Amonyl 380BA (CAPB) Cocylamidopropylbetaine | 2.00 |
| Jaguar C-13-S | 0.25 |
| NaCl | 0.30 |
| Water and minors | to 100% |

### Preparation

The glycerol, sucrose ester, stearic acid and cholesterol were melted together on a hot plate. The surfactants were premixed and added to the excess water. The surfactants mixture was then added to the lipid/glycerol mixture followed by addition of the Jaguar. Preservative was added and the pH adjusted to 5.3. The viscosity was adjusted to 5000 mPas at 10s⁻¹/25°C using NaCl.

### Example 13. Deposition of Cholesterol on skin

Deposition of cholesterol onto pig skin from the liquid cleanser compositions was determined by the following method. 0.25 grams of cleanser was rubbed 50 times onto a piece (5 x 5 cm) of pig skin prewetted with tap water. The skin was then rinsed for 10 seconds with deionized water and patted dry with a paper towel. 3 ml of ethanol was used to extract the cholesterol from the skin for analysis.

The amount of cholesterol extracted from the skin was determined by a spectrophotometric method. The ethanol extraction was dried at 80°C oven. 100 µl methanol was added to the dried content. 1,000 µl aqueous cholesterol reagent (ex. Sigma) was added to the sample and left for 5 minutes to react with the extracted cholesterol. The aqueous cholesterol solution was then mixed with 500 µl chloroform and poured into microcentrifuge tubes. Chloroform was separated from the aqueous solution by cetrifuging the tubes at 13,000 rpm for 5 minutes. After centrifugation the top clear aqueous layer was pipetted into curvets and the absorbance at 500 nm was measured using a spectrophotometer and a standard calibration curve to determine the amount of cholesterol extracted from the skin.

The amount of cholesterol extracted from the skin treated with the composition of Example 12 was compared with two controls in Table 10. The data shows that significant deposition of cholesterol was achieved from the hydrogel sample of Example 12 as compared to the two controls.

**Table 10.**

| **Deposition of Cholesterol from shower gel** | |
|---|---|
| Sample No. | Deposition of cholesterol µg/10 cm² |
| Example 12 | 48.4 |
| Control 1^{a} | 26.5 |
| Control 2^{b} | 21.1 |

| | |
|---|---|
| a. Control 1 is liquid cleanser which contained 2.5% by wt. of dispersed cholesterol. The composition and preparation are given in Table 9. | |
| b. Control 2 is a liquid cleanser without any cholesterol. (The amount of cholesterol given in the table is the cholesterol extracted from the pig skin). | |

### Example 14

A liquid cleanser composition containing 10% by wt. of silicone hydrogel (5% by wt. silicone oil of the total composition) and 90% by wt. of surfactant composition of Table 3 with 0.3% by wt. instead of 0.97% Antil 141 was prepared using the process described in Example 3. The silicone hydrogel precursor solution containing 50% by wt. 60,000 cps silicone oil, 0.47% by wt. Carrageenan GP911, 0.155% by wt. Aculyn-33, 0.31% by wt. polyvinyl alcohol (Airvol 540 ex Air Product) and 24.06% by wt. water was prepared as described in Table 3. The hydrogel precursor solution contained silicone oil droplets with size in the range of 5 to 60 µm.

The silicone hydrogel containing cleanser was compared to the liquid cleanser without silicone hydrogel dispersion using the protocol described below. The test site for this evaluation is the volar surface of the forearm. Hand and forearm were prewetted by holding them under running tap water (30°C) for approximately 15 seconds. 3.5 grams of the hydrogel containing liquid cleanser was then dispensed onto the hand and the assigned arm was washed for 20 seconds and rinsed under running water for 10 seconds. The forearm was patted dry with a paper towel. The procedure was repeated for the other arm using the same cleanser without containing the silicone hydrogel. All panellists were asked to determine their perception of after use skin feel. 7 out of the 8 panellists felt a difference in after-use skin feel and 5 out of those 7 preferred the skin feel of the arm treated with the silicone hydrogel containing liquid cleanser.

### Example 15: Hydrogel Dispersion in Soap Base Liquids

A liquid soap base of formulation given in Table 11 was used for this example. A hydrogel precursor was made by mixing 100 grams of 60,000 cps silicone oil to 100 grams of hydrogel solution containing 2% by wt. carrageenan GP911, 1% by wt. of Aculyn 33 and 2% by wt. of polyvinyl alcohol using an overhead mixer. The prepared hydrogel precursor contained silicone oil droplets with size in the range 2 µm to 80 µm. The extrusion process described in Example 3 was then used to make the soap base liquid cleanser containing 10% by wt. of the silicone hydrogel stably dispersed in the soap base liquid cleanser. This liquid cleanser had very good lather during use and was perceived as leaving the skin with a very silky smooth feel after use.

**Table 11**

| Ingredient | % by wt. |
|---|---|
| Oleic Acid | 6.8 |
| Lauric Acid | 5.8 |
| Myristic Acid | 3.4 |
| Palmitic Acid | 1.0 |
| Potassium Hydroxide | 4.0 |
| Cocamido Propyl Betaine | 5.0 |
| Laponite XLS* | 1.5 |
| Perfume | 1.0 |
| Water | to 100 |
| pH | 9.2 |
| Viscosity | 7,800 cps at 10 rps |

| | |
|---|---|
| * Smectite clay used for thickening. | |

## Claims

1. A liquid cleanser composition comprising:
(1) 1 to 60% by wt. of an aqueous surfactant solution comprising at least one surfactant selected from anionic, nonionic, cationic, zwitterionic and amphoteric surfactants and mixtures thereof; and
(2) 1 to 50% by wt. of a hydrogel composition comprising:
(a) 0.5 to 50% by wt., based on the hydrogel composition, of at least one first water-soluble polymer which is insolubilized in said aqueous surfactant solution;
(b) 0.2 to 25% by wt., based on the hydrogel composition, of at least one second water-soluble polymer which is either soluble or dispersible in said aqueous surfactant solution; and
(c) 5 to 65% by wt., based on the hydrogel composition, of a water-insoluble cosmetic benefit agent which is entrapped in a network formed by said first and second polymers, said benefit agent having a particle size in the range 0.2 to 200 µm; and
said hydrogel composition comprising particles of a size with at least one dimension in the range of more than 25 and up to 10,000 µm.

2. A liquid cleanser according to claim 1 where the first water-soluble polymer is made insoluble when contacted with said aqueous surfactant solution by a process selected from:
(a) thermal gelation;
(b) precipitation;
(c) coacervation; and
(d) cross-linking.

3. A liquid cleanser according to claim 2 wherein the first polymer is formed by thermal gelation and is water-soluble at a temperature higher than 40-50°C and forms a gel when cooled to room temperature.

4. A liquid cleanser according to claim 3 wherein the first polymer is selected from carrageenan and gelatin.

5. A liquid cleanser according to claim 2 wherein the first polymer is formed by precipitation, coacervation or cross-linking, is water-soluble at room temperature and is made substantially insoluble by changing the physical or chemical properties of the aqueous surfactant solution with which said polymer is contacted.

6. A liquid cleanser according to claim 5 wherein the first polymer is selected from polyglucosamine; polyvinylalcohol having molecular weight greater than 13,000 Daltons and a degree of hydrolysis in the range of about 78% to 100%; and hydroxycelluloses.

7. A liquid cleanser according to claim 5 wherein the first polymer forms a water-insoluble complex with a water-soluble cross-linker.

8. A liquid cleanser according to claim 1, wherein the second polymer is selected from carboxylic acid containing acrylic polymers; nonionic polymers selected from polyvinyl alcohol, polyvinyl pyrridone, modified corn starch and hydroxyethylcellulose; cationic polymers selected from cationic guar, cationic cellulose, synthetic cationic polymers; and vinyl pyrrolidene/dimethamine ethyl methacrylate copolymer.

9. A liquid cleanser composition according to claim 1, wherein the cosmetic benefit agent is selected from silicone oils; silicone and organosilicone oils and gums; fats and oils; waxes; hydrophobic plant extracts; hydrocarbons; C₁₂ to C₃₀ saturated and unsaturated fatty acids; C₁₂ to C₃₀ alcohols; esters; essential oils; lipids; vitamins; germicides; sunscreens; derivatives of alpha hydroxy acids and mixtures thereof.

10. A liquid cleanser composition according to claim 1 prepared by first mixing said water-insoluble cosmetic benefit agent with a solution of said first and second polymers and subsequently mixing with said aqueous surfactant solution.

## Patentansprüche

1. Eine Flüssigreiniger-Zusammensetzung, enthaltend:
(1) 1 bis 60 Gew.-% einer wässerigen Tensid-Lösung, enthaltend zumindest ein Tensid, ausgewählt aus anionischen, nichtionischen, kationischen, zwitterionischen und amphoteren Tensiden und Mischungen derselben; und
(2) 1 bis 50 Gew.-% einer Hydrogel-Zusammensetzung, enthaltend:
(a) 0,5 bis 50 Gew.-%, basierend auf der Hydrogel-Zusammensetzung, von zumindest einem ersten wasserlöslichen Polymeren, welches in der erwähnten wässerigen Tensid-Lösung unlöslich gemacht ist;
(b) 0,2 bis 25 Gew.-%, basierend auf der Hydrogel-Zusammensetzung, von zumindest einem zweiten wasserlöslichen Polymeren, welches in der erwähnten wässerigen Tensid-Lösung entweder löslich oder dispergierbar ist; und
(c) 5 bis 65 Gew.-%, basierend auf der Hydrogel-Zusammensetzung eines wasserunlöslichen kosmetisch vorteilhaften Mittels, welches in einem Netzwerk eingeschlossen ist, gebildet durch die erwähnten ersten und zweiten Polymeren, wobei das erwähnte vorteilhafte Mittel eine Teilchengröße in dem Bereich von 0,2 bis 200 µm hat; und
wobei die erwähnte Hydrogel-Zusammensetzung Teilchen einer Größe mit zumindest einer Dimension in dem Bereich von mehr als 25 und bis zu 10,000 µm enthält.

2. Ein Flüssigreiniger nach Anspruch 1, worin das erste wasserlösliche Polymere unlöslich gemacht ist, wenn in Kontakt mit der erwähnten wässerigen Tensid-Lösung durch ein Verfahren, ausgewählt aus :
(a) Thermischer Gelatinierung;
(b) Ausfällung;
(c) Flockenbildung; und
(d) Vernetzung.

3. Ein Flüssigreiniger nach Anspruch 2, worin das erste Polymere durch thermische Gelatinierung gebildet und bei einer Temperatur von höher als 40° bis 50°C wasserlöslich ist und ein Gel bildet, wenn auf Raumtemperatur abgekühlt ist.

4. Ein Flüssigreiniger nach Anspruch 3, worin das erste Polymere aus Carrageenan und Gelatine ausgewählt ist.

5. Ein Flüssigreiniger nach Anspruch 2, worin das erste Polymere durch Ausfällung, Flockenbildung oder Vernetzung gebildet ist, bei Raumtemperatur wasserlöslich ist und im wesentlichen unlöslich durch Änderung der physikalischen oder chemischen Eigenschaften der wässerigen Tensid-Lösung gemacht ist, mit welcher das erwähnte Polymere in Kontakt gebracht ist.

6. Ein Flüssigreiniger nach Anspruch 5, worin das erste Polymere ausgewählt ist aus Polyglucosamin; Polyvinylalkohol mit einem Molekulargewicht von größer als 13,000 Dalton und einem Hydrolysegrad in dem Bereich von etwa 78% bis 100%; und Hydroxycellulosen.

7. Ein Flüssigreiniger nach Anspruch 5, worin das erste Polymere einen wasserunlöslichen Komplex mit einem wasserlöslichen Vernetzer bildet.

8. Ein Flüssigreiniger nach Anspruch 1, worin das zweite Polymere aus Carbonsäure ausgewählt ist, enthaltend Acrylpolymere; nichtionische Polymere, ausgewählt aus Polyvinylalkohol, Polyvinylpyrridon, modifizierter Maisstärke und Hydroxyethylcellulose; kationische Polymere, ausgewählt aus kationischem Guargummi, kationischer Cellulose, synthetischen kationischen Polymeren; und Vinylpyrroliden/Dimethaminethylmethacrylat-Copolymeres.

9. Eine Flüssigreiniger-Zusammensetzung nach Anspruch 1, worin das kosmetisch vorteilhafte Mittel ausgewählt ist aus Silicon-Ölen; Silicon- und Organosilicon-Ölen und -Gummis; Fetten und Ölen; Wachsen; hydrophoben Pflanzenextrakten; Kohlenwasserstoffen; gesättigten und ungesättigten C₁₂₋₃₀-Fettsäuren; C₁₂₋₃₀-Alkoholen; Estern; wesentlichen Ölen; Lipiden; Vitaminen; Germiciden; Sonnenschutzmitteln; Derivaten von α-Hydroxysäuren und Mischungen derselben.

10. Eine Flüssigreiniger-Zusammensetzung nach Anspruch 1, hergestellt durch erstes Mischen der erwähnten wasserunlöslichen, kosmetisch vorteilhaften Mittel mit einer Lösung der erwähnten ersten und zweiten Polymeren und anschließendem Mischen mit der erwähnten wässerigen Tensid-Lösung.

## Revendications

1. Composition nettoyante liquide comprenant :
(1) de 1 à 60 % en masse d'une solution aqueuse d'agents tensioactifs comprenant au moins un agent tensioactif sélectionné à partir des agents tensioactifs anioniques, non ioniques, cationiques, zwitterioniques et amphotères, et des mélanges de ceux-ci ; et
(2) de 1 à 50 % en masse d'une composition d'hydrogel comprenant :
(a) de 0,5 à 50 % en masse, sur la base de la composition d'hydrogel, d'au moins un polymère soluble dans l'eau qui n'est pas solubilisé dans ladite solution aqueuse ;
(b) de 0,2 à 25 % en masse, sur la base de la composition d'hydrogel, d'au moins un second polymère soluble dans l'eau qui est soit soluble, soit dispersible dans ladite solution aqueuse d'agents tensioactifs ; et
(c) de 5 à 65 % en masse, sur la base de la composition d'hydrogel, d'un agent insoluble dans l'eau et bénéfique du point de vue cosmétique, qui est emprisonné dans un réseau formé par lesdits premiers et seconds polymères, ledit agent bénéfique ayant une taille de particule comprise dans la gamme allant de 0,2 à 200 micromètres ; et
ladite composition d'hydrogel comprenant des particules ayant une taille telle qu'au moins l'une de ses dimensions est comprise dans la gamme allant de plus de 25 micromètres jusqu'à 10.000 micromètres.

2. Nettoyant liquide selon la revendication 1, dans lequel le polymère soluble dans l'eau est rendu insoluble lorsqu'il est mis en contact avec ladite solution aqueuse d'agents tensioactifs, et ce par un procédé sélectionné parmi :
(a) le gel thermique ;
(b) la précipitation ;
(c) la co-acervation ; et
(d) la réticulation

3. Nettoyant liquide selon la revendication 2, dans lequel le premier polymère est formé par gel thermique et est soluble dans l'eau à une température supérieure à 40 - 50°C et forme un gel lorsqu'il est refroidi à température ambiante.

4. Nettoyant liquide selon la revendication 3 dans lequel le premier polymère est sélectionné à partir du carragheenane et la gélatine.

5. Nettoyant liquide selon la revendication 2 dans lequel le premier polymère est formé par précipitation, co-acervation ou réticulation, est soluble dans l'eau à température ambiante et est rendu substantiellement non soluble par changement des propriétés physiques ou chimiques de la solution aqueuse d'agents tensioactifs avec laquelle ledit polymère est mis en contact.

6. Nettoyant liquide selon la revendication 5, dans lequel le premier polymère est sélectionné à partir du polyglucosamine ; de l'alcool polyvinylique ayant une masse moléculaire supérieure à 13.000 Daltons et un niveau d'hydrolyse compris dans la gamme allant d'environ 78 % à 100 %; et de l'hydroxycellulose.

7. Nettoyant liquide selon la revendication 5, dans lequel le premier polymère forme un complexe non soluble dans l'eau avec un agent de réticulation soluble dans l'eau.

8. Nettoyant liquide selon la revendication 1, dans lequel le second polymère est sélectionné à partir des polymères acryliques contenant de l'acide carboxylique ; des polymères non ioniques sélectionnés à partir de l'acide polyvinylique, de la pyrridone polyvinylique, de l'amidon de maïs modifié et de l'hydroxyéthylcellulose ; des polymères cationiques sélectionnés à partir de la gomme de guar, de la cellulose cationique, des polymères cationiques synthétiques ; et le copolymère de pyrrolidène vinylique et de méthacrylate de diméthamine éthylique.

9. Composition nettoyante liquide selon la revendication 1, dans laquelle l'agent bénéfique du point de vue cosmétique est sélectionné à partir des huiles de silicone ; des huiles et des gommes de silicone et d'organosilicone ; des graisses et des huiles; des cires; des extraits de plantes hydrophobes; des hydrocarbures ; des acides gras en C₁₂ à C₃₀ saturés et insaturés ; des alcools en C₁₂ à C₃₀ ; des huiles essentielles ; des lipides ; des vitamines ; des germicides; des écrans solaires ; des dérivés d'acides alpha hydroxy et des mélanges de ceux-ci.

10. Composition nettoyante liquide selon la revendication 1, préparée en mélangeant premièrement ledit agent bénéfique d'un point de vue cosmétique non soluble dans l'eau et ensuite en mélangeant cela avec la solution aqueuse d'agents tensioactifs.
